# EUROPEAN PATENT APPLICATION

(11) **EP 4 800 099 A1**
(43) Date of publication of application: **02.09.2026**
(21) Application number: 24882347.8
(22) Date of filing: 21.10.2024
(51) Int. Cl.: C12N 1/00, C12M 1/00

(54) **METHOD FOR PRODUCING CELL PROCESSED PRODUCT AND DEVICE FOR PRODUCING CELL PROCESSED PRODUCT**

(30) Priority: 23.10.2023 JP 2023181827
(71) Applicant: Daicel Corporation, Osaka-shi, Osaka 530-0011 (JP)
(72) Inventor: ATOBE, Shingo, Tokyo 108-8230 (JP); SHIGEMATSU, Masato, Tokyo 108-8230 (JP)
(74) Representative: Grünecker Patent- und Rechtsanwälte PartG mbB
(86) International application number: PCT/JP2024/037428
(87) International publication number: WO 2025/089234

(57) **Abstract**

An object of the present disclosure is to provide a novel method that achieves improvement in cell permeability without cell disruption. According to the method for producing a cell-processed product of the present disclosure, partial damage is inflicted on the surface layer of a cell by applying a pressure wave to the cell with a predetermined pressure profile using a liquid as a pressure wave medium, and thus, improvement in cell permeability is achieved without cell disruption.

## Description

### Technical Field

The present disclosure relates to a method for producing a cell-processed product and an apparatus for producing a cell-processed product.

### Background Art

As one form of cell processing methods, a method using pressure has been known. Such a method is generally referred to as high hydrostatic pressure processing (HHP) or ultra-high pressure processing (UHP). These methods aim to kill single cells, such as food poisoning bacteria and spoilage bacteria, in food, and specifically, pressure conditions in which a pressure of several hundred MPa is applied for several ten seconds to several hundred seconds are used (Non-Patent Literature 1). Such pressure conditions cause a phase transition of relatively fluid cell membranes and/or cell walls, and thus, the cell membranes and/or the cell walls are hardened and/or fragilized. As a result, cell surface layers are disrupted, leading to cell death.

Cell disruption can be used not only for applications in food sterilization as described above, but also for applications in molecular diagnostics (e.g., pathogen detection platforms, immunoassays for Point of Care Testing, protein purification for studying the function and/or the structure of protein, cancer diagnostics, drug screening, mRNA transcriptome determination, and composition analysis of specific proteins, lipids, and/or nucleic acids) by obtaining intracellular materials such as DNA, RNA, protein, or organelles from cells. To achieve efficient cell disruption even in applications with a small processing scale such as molecular diagnostics, unlike food sterilization applications, a cell disruption apparatus including a pressure chamber pressurized using an explosive has been proposed (Patent Document 1).

On the other hand, as other forms of the cell processing methods, electroporation and sonoporation have also been known. Unlike the above method using pressure, these methods are used for delivering a drug or gene into cells while suppressing damage to the cells.

### Citation List

### Non-Patent Literature

Non-Patent Literature 1: Bon Kimura, "Shokuhin Biseibutsugaku (Kensa to Seigyo Hoho), Kiso to Saishin Joho wo Kaisetsu, Shokuhin no Koatsu Shori Sakkin", [online], Apr. 11, 2022, [retrieved on Oct. 9, 2023], Internet <URL: https://foodmicrob.com/high-pressure-sterilization/>

### Patent Document

Patent Document 1: WO 2021/085491

### Summary of Invention

### Technical Problem

In known cell processing methods, when the purpose of the methods involves cell disruption, pressure may be selected as a processing means, and when the purpose of the methods does not involve cell disruption, electricity or ultrasonic waves may be selected as a processing means. The latter processing means improves cell permeability by inflicting partial damage on the surface layer of cells without disrupting the cells, and thus, the targeted processing effects are achieved.

In order to process cells without disrupting the cells, it is desirable that damage inflicted on the cells is as small as possible. On the other hand, there is a trade-off relationship between suppression of the damage inflicted on cells and improvement in cell processing effects, that is, improvement in cell permeability. Thus, it is common technical knowledge that both of the suppression and the improvement cannot be improved in principle. In this respect, the electric or ultrasonic processing method has a limit as a method for improving cell permeability without cell disruption.

In view of such background, an object of the present disclosure is to provide a novel method that achieves improvement in cell permeability without cell disruption.

### Solution to Problem

As a result of intensive studies, the present inventors have found that improvement in cell permeability is unexpectedly achieved without cell disruption by daringly adopting a pressure means that has been heretofore used for cell disruption and applying a pulse pressure wave with a predetermined pressure profile using a liquid as a medium. Further, unexpectedly, it has also been found that damage to the surface layer of a cell by the above means is suppressed as compared with electroporation, and at the same time, the amount of a cytoplasmic component released through the cell permeation can be increased. The present disclosure has been completed through further examinations based on these findings.

The present disclosure provides the following aspects.
Item 1. A method for producing a cell-processed product, the method including
   processing a cell by applying a pressure wave to the cell using a liquid as a pressure wave medium, and thus, inflicting partial damage on a surface layer of the cell, wherein
   a pressure profile of the pressure wave includes conditions (i) to (iii):
      (i) a maximum value of the pressure is 37 MPa or greater and 500 MPa or less;
      (ii) time from start of the pressure rise to a time point when the pressure reaches the maximum value is 0.5 ms or less; and
      (iii) time from start of the pressure decay from the maximum value to a time point when the pressure decays to 1/2 of the maximum value is 5 ms or less.
Item 2. The production method according to Item 1, wherein
   the pressure wave is applied to a container containing a first liquid with the cell, using a second liquid as the pressure wave medium, the container being immersed in the second liquid.
Item 3. The production method according to Item 1 or 2, wherein
   the cell coexists with a molecule to be introduced into the cell, and in the processing the cell, the molecule is introduced into the cell through the partial damage; and
   the cell-processed product is the cell containing the introduced molecule.
Item 4. The production method according to Item 3, wherein
   the molecule is selected from the group consisting of a cell-staining dye, a drug delivery carrier, and a drug delivery carrier labeled with the cell-staining dye.
Item 5. The production method according to Item 1 or 2, wherein
   in the processing the cell, at least a part of a cytoplasmic component of the cell is extracellularly released through the partial damage; and
   the cell-processed product is a cell lacking the at least a part of the cytoplasmic component and/or the at least a part of the cytoplasmic component.
Item 6. The production method according to any one of Items 1 to 5, wherein
   the application is repeatedly operated in the processing the cell.
Item 7. An apparatus for producing a cell-processed product, the apparatus including:
   a liquid as a pressure wave medium;
   a pressure-resistant container configured to accommodate a cell;
   a pressure wave application unit configured to apply a pressure wave into the pressure-resistant container through the liquid with a pressure profile, the pressure profile including conditions (i) to (iii):
      (i) a maximum value of the pressure is 37 MPa or greater and 500 MPa or less;
      (ii) time from start of the pressure rise to a time point when the pressure reaches the maximum value is 0.5 ms or less; and
      (iii) time from start of the pressure decay from the maximum value to a time point when the pressure decays to 1/2 of the maximum value is 5 ms or less.
Item 8. The production apparatus according to Item 7, wherein
   the pressure wave application unit is not loaded with a gas generating agent.

### Advantageous Effects of Invention

According to the present disclosure, a novel method that achieves improvement in cell permeability without cell disruption is provided.

### Brief Description of Drawings

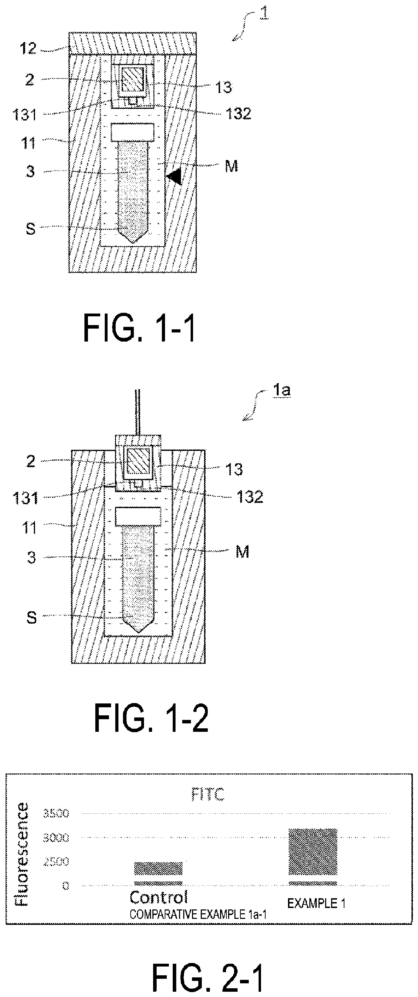

FIG. 1-1 illustrates a schematic cross-sectional view of an embodiment of a cell-processed product production apparatus of the present disclosure.
FIG. 1-2 illustrates a schematic cross-sectional view of another embodiment of the cell-processed product production apparatus of the present disclosure.
FIG. 2-1 shows evaluation results obtained using a fluorescence plate reader for Comparative Example 1a-1 and Example 1.
FIG. 2-2 shows evaluation results obtained using a flow cytometer for Comparative Example 1a-1 and Example 1.
FIG. 3-1 shows evaluation results obtained using a fluorescence plate reader for Comparative Example 1a-2 and Example 2.
FIG. 3-2 shows evaluation results obtained using a flow cytometer for Comparative Example 1a-2 and Example 2.
FIG. 4-1 shows evaluation results obtained using a fluorescence plate reader for Comparative Example 1a-3 and Example 3.
FIG. 4-2 shows fluorescence microscopic images obtained in Comparative Example 1a-3 and Example 3.
FIG. 5-1 shows an SEM image obtained in Comparative Example 1b.
FIG. 5-2 shows an SEM image obtained in Example 4a.
FIG. 5-3 shows an SEM image obtained in Comparative Example 2.
FIG. 6 is an electrophoretic image of extracts according to Example 4b and Comparative Examples 3a to 3e.
FIG. 7-1 shows the evaluation results of viable cell count for Comparative Example 1a-1 and Example 1.
FIG. 7-2 shows the evaluation results of viable cell count for Comparative Example 1a-2 and Example 2.
FIG. 7-3 shows the evaluation results of viable cell count for Comparative Example 1a-3 and Example 3.
FIG. 8 shows the evaluation results of viable cell count for Comparative Example 1c, Example 4b, Comparative Example 3c, and Comparative Example 4.

### Description of Embodiments

### 1. Method for Producing Cell-Processed Product

A method for producing a cell-processed product of the present disclosure includes processing a cell by applying a pressure wave to the cell with a predetermined pressure profile using a liquid as a pressure wave medium, and thus, inflicting partial damage on a surface layer of the cell. Hereinafter, the method for producing a cell-processed product of the present disclosure will be described in detail. Note that, in the present specification, a numerical range indicated by two numerical values and the word "to" shall be understood to include the two numerical values as the lower limit value and the upper limit value. For example, the expression "from 2 to 15 wt.%" means 2 wt.% or greater and 15 wt.% or less.

### 1-1. Cell-Processed Product

The cell-processed product in the present disclosure is not particularly limited as long as it is a resultant product obtained by the production method of the present disclosure, and typical examples thereof include at least any one of a cell into which an exogenous molecule (hereinafter, also simply referred to as a "molecule") is introduced, a cell lacking at least a part of a cytoplasmic component of the cell, and a cytoplasmic component released from the cell.

The type of the cell is not particularly limited, and may be any of a prokaryotic cell (e.g., bacteria such as Escherichia coli, lactic acid bacteria, and Bacillus subtilis var. natto) and a eukaryotic cell. The type of the eukaryotic cell is also not particularly limited, and examples thereof may be any of plant cells (e.g., angiosperm cells, gymnosperm cells, and algal cells); animal cells (e.g., human cells, non-human mammalian cells, avian cells, reptile cells, amphibian cells, fish cells, and insect cells); and microbial cells (e.g., yeast, and filamentous fungi).

The molecule is not particularly limited, and examples thereof include a cell-staining dye, a drug delivery carrier, and a drug delivery carrier labeled with the cell-staining dye. A single type of these molecules may be used alone, or a plurality of types thereof may be used in combination.

Examples of the cell-staining dye may be a dye that stains any part of a cell, and include fluorescent dyes having a positive charge [specifically, propidium iodide (PI), ethidium bromide (EB), 4',6-diamidino-2-phenylindole (DAPI), and acridine orange (AO), for example], and fluorescein-based dyes having an amino group-reactive group (e.g., an isothiocyanate group, and a succinimidyl group) as a substituent [specifically, fluorescein isothiocyanate (FITC) and fluorescein succinimidyl ester, for example]. Among these, fluorescent dyes having a positive charge generally do not permeate or hardly permeate viable cells, but according to the production method of the present disclosure, even in a fluorescent dye having a positive charge, the dye can be effectively introduced into a viable cell.

Examples of the drug delivery carrier include biocompatible polymers, more specifically polysaccharides such as dextran, pullulan, arabinogalactan, and mannan, and synthetic polymers such as polyvinyl alcohol. The size of the drug delivery carrier is not particularly limited, but examples thereof include from 1000 Da to 500000 Da, preferably from 2000 Da to 50000 Da, more preferably from 3000 Da to 10000 Da, and still more preferably from 3500 Da to 5000 Da. Examples of the weight-average molecular weight of the drug delivery carriers include from 1000 to 500000, preferably from 2000 to 50000, more preferably from 3000 to 10000, and still more preferably from 3500 to 5000. Note that the weight-average molecular weight in the present disclosure is a value converted to polystyrene standard as measured by gel permeation chromatography.

The cytoplasmic component is not particularly limited, and examples thereof include proteins, sugar chains, and nucleic acids.

### 1-2. Pressure Wave Medium

In the production method of the present disclosure, a liquid is used as the pressure wave medium for carrying out the predetermined pressure profile. The type of the liquid is not particularly limited, and examples thereof include water, an aqueous solution, an organic solvent, an ionic liquid, and a gel-like liquid. A single type of these liquids may be used alone, or two or more types thereof may be used in combination. Among these liquids, water or a liquid having a bulk modulus close to that of water (e.g., a bulk modulus at 25°C and 1 atm of from 0.5 to 5 GPa, preferably from 0.5 to 3 GPa, and more preferably from 0.5 to 1 GPa) is preferable, and water is particularly preferable.

The amount of the pressure wave medium used is not particularly limited, but examples thereof include from 3 to 100 ml, preferably from 5 to 50 ml, and more preferably from 7 to 15 ml.

### 1-3. Cell to be Processed

The type of the cell to be processed is as described in "1-1. Cell-Processed Product" above.

The concentration of the cell to be processed is not particularly limited, but examples thereof include from 1 × 10⁷ to 1 × 10¹² cells/mL, preferably from 1 × 10⁸ to 1 × 10¹¹ cells/mL, and more preferably from 5 × 10⁸ to 5 × 10¹⁰ cells/mL.

The cells are provided in a state of being contained in a liquid. The liquid may be shared with the liquid used as the pressure wave medium or may be physically separated from the liquid used as the pressure wave medium. When the liquid is physically separated from the liquid used as the pressure wave medium, specifically, a container containing a first liquid with the cell (specifically, a cell suspension) can be provided in a form of being immersed in a second liquid as the pressure wave medium. The material of the container containing the first liquid with the cell is not particularly limited, but a material having an acoustic impedance close to that of the medium is desirable because such a material does not interfere with the pressure wave. Examples of the material include silicone resin and fluororesin (e.g., perfluoroalkoxyalkane polymer (PFA), and perfluoroethylene-propene copolymer (FEP)).

As the liquid for incorporating the cell, water is typically used. The liquid may contain an optional component in addition to the cell. Specific examples of the optional component include a tonicity agent, a buffer, and a molecule for introduction into cells. A single type of these optional components may be used alone, or two or more types thereof may be used in combination.

### 1-4. Application of Pressure Wave

The pressure wave is applied to the cell through the pressure wave medium. The means for applying the pressure wave is not particularly limited, but generally, a means for impulsively generating a pressure wave in liquid (dynamic pressure wave) is used, and specific examples thereof include actuation of a pyrotechnic device, drop of a heavy object, collision of a projectile, rapid compression by a spring, gas, or electromagnetic force, and a gas-driven shock tube.

The pyrotechnic device preferably generates a propulsive explosion that transmits a propulsive force due to thermal expansion at a speed equal to or less than the speed of sound, and contains an explosive as a substance that produces a propulsive force due to thermal expansion by burning at a speed equal to or less than the speed of sound. Specific examples of the pyrotechnic device include an actuator including an igniter (that is, an initiator) in which an ignition charge (pyrotechnic charge) is loaded in an outer shell made of, for example, metal. A gas generating agent is not loaded in the actuator. In this actuator, an electrical signal supplied from a power source is applied to the initiator, and thus, the ignition charge loaded in the initiator is ignited. As the temperature inside the igniter becomes high, the outer shell thereof is opened, the actuator is released, and the plasma-like flame thus emitted compresses the pressure medium to generate a pressure wave, which propagates through the pressure wave medium. Examples of the ignition charge include an explosive containing zirconium and potassium perchlorate (ZPP), an explosive containing titanium hydride and potassium perchlorate (THPP), an explosive containing titanium and potassium perchlorate (TiPP), an explosive containing aluminum and potassium perchlorate (APP), an explosive containing aluminum and bismuth oxide (ABO), an explosive containing aluminum and molybdenum oxide (AMO), an explosive containing aluminum and copper oxide (ACO), and an explosive containing aluminum and iron oxide (AFO). The ignition charge may be one type of these explosives alone, or two or more types thereof may be used in combination. Among these explosives, ZPP is preferable.

The distance between a pressure wave application unit (pressure wave generation unit, corresponding to a breakable portion 132 forming an outlet of a pressure relief channel 131 at the time of driving in an apparatus 1, 1a for producing a cell-processed product as described later) and the cell is not particularly limited, and may vary depending on the processing scale, but the shortest distance between the application unit and the cell can be set to, for example, from 10 to 300 mm, preferably from 15 to 200 mm, more preferably from 15 to 100 mm, and still more preferably from 15 to 60 mm or from 15 to 40 mm.

The application operation may be performed once or a plurality of times, and is preferably performed a plurality of times. The specific number of times of the application operation may vary depending on the type of the cell, but the application operation can be performed, for example, four times or more, more preferably 10 times or more, and still more preferably 14 times or more. The upper limit of the specific number of times of the application operation is also not particularly limited, and examples thereof include 40 times or less, 20 times or less, and 18 times or less. Specific examples of the range of the number of times include from 4 to 40 times, preferably from 10 to 20 times, and more preferably from 14 to 18 times. The above examples of the number of times are preferably applied, when the cell is yeast, for example.

### 1-5. Pressure Profile

The pressure profile of the pressure wave includes conditions (i) to (iii):
(i) the maximum value of the pressure is 37 MPa or greater and 500 MPa or less;
(ii) time from start of the pressure rise to a time point when the pressure reaches the maximum value is 0.5 ms or less; and
(iii) time from start of the pressure decay from the maximum value to a time point when the pressure decays to 1/2 of the maximum value is 5 ms or less.

The preferable range of the maximum value in the condition (i) may vary depending on the type of the cell, and examples thereof include from 37 MPa to 450 MPa, preferably from 60 MPa to 400 MPa, more preferably from 80 MPa to 350 MPa, still more preferably from 100 MPa to 300 MPa, and even more preferably from 130 MPa to 280 MPa. The above examples regarding the preferable range of the maximum value in the condition (i) are preferably applied, when the cell is yeast, for example. Examples of the preferable range of the time in the condition (ii) include from 0.01 to 0.4 ms, preferably from 0.02 to 0.3 ms, more preferably from 0.03 to 0.2 ms, and still more preferably from 0.035 to 0.15 ms. Examples of the preferable range of the time in the condition (iii) include from 0.1 to 3 ms, preferably from 0.12 to 1.5 ms, more preferably from 0.14 to 0.8 ms, and still more preferably from 0.16 to 0.4 ms.

Note that the measurement location of the above profile is set based on the distance between the pressure wave application unit and the measurement location. This distance is about from one to two times the shortest distance between the pressure wave application unit and the location where the cell exists. When the measurement is performed at a location within this range, a comparable measurement value can be obtained. Note that, when a highly directional pressure wave is used as a power source, the measurement may be performed in consideration of a direction in which the pressure wave front can be received.

The above pressure profile can be carried out by using a liquid as the pressure wave medium and outputting a pressure wave under the above conditions (i) and (ii) in the above liquid.

Further, the maximum value in the above condition (i) can be controlled by adjusting the driving force level of the means for applying the pressure wave, and by the distance between the target to be applied and the driving source, for example. For example, when a pyrotechnic device is used as the means for applying the pressure wave, the maximum value in the above condition (i) can be controlled by adjusting the amount of the ignition charge contained in the pyrotechnic device, and when drop of a heavy object is used as the means for applying the pressure wave, the maximum value in the above condition (i) can be controlled by adjusting the weight of the object to be dropped or the drop distance. More specifically, examples of the amount of the ignition charge used include from 50 to 300 mg, and preferably from 90 to 240 mg. In addition, the time in the above conditions (ii) and (iii) can be controlled by, for example, adjusting the burning rate of the ignition charge when a pyrotechnic device is used. The above distance is as described in "1-4. Application of Pressure Wave" above.

### 1-6. Infliction of Partial Damage on Surface Layer of Cell

Partial damage is inflicted on the surface layer of the cell by applying a pressure wave to the cell with the predetermined pressure profile using the liquid as the pressure wave medium. The partial damage means that damage (examples of specific forms of the damage include pores, and fluctuations in an arrangement of molecules contained in a molecular assembly) that allows communication between the intracellular space and the extracellular space is formed in the surface layer of the cell, and the formation of the damage allows permeation of molecules that normally do not permeate through viable cells, while the degree of the damage is minute to the extent that the cell is not disrupted and that the shape of the cell is substantially maintained (that is, the cell is maintained in a form that does not cause cell death). The partial damage can be confirmed by introduction of an exogenous molecule into the cell and the survival of the cell.

When the cell into which an exogenous molecule is introduced is produced as the cell-processed product, the molecule is introduced into the cell through partial damage by inflicting the partial damage on the surface layer of the cell.

When a cell lacking at least a part of a cytoplasmic component and/or a cytoplasmic component released from the cell are produced as the cell-processed product, at least a part of the cytoplasmic component of the cell is extracellularly released through the partial damage by inflicting the partial damage on the surface layer of the cell. The morphology of the cell thus obtained is not disrupted, and the cell only lacks at least a part of the cytoplasmic component. Therefore, the cell can be used again for another application. In particular, when the cell is rare, for example, it is highly useful to yield the cell in such a morphology. In addition, the fact that a cytoplasmic component is obtained without disrupting the cell in this manner is particularly advantageous in terms of recovery efficiency because it does not involve contamination with a large amount of unnecessary cell components (e.g., cell membrane components, and organelles).

### 2. Apparatus for Producing Cell-Processed Product

The apparatus for producing a cell-processed product of the present disclosure includes: a liquid as a pressure wave medium; a pressure-resistant container configured to accommodate a cell; and a pressure wave application unit configured to apply a pressure wave into the pressure-resistant container through the liquid with a predetermined pressure profile, the apparatus lacking a pressure relief hole in communication with the internal space and the external space of the pressure-resistant container. The apparatus for producing a cell-processed product of the present disclosure can be used for carrying out "1. Method for Producing Cell-Processed Product" above.

FIGS. 1-1 and 1-2 illustrate schematic views of an embodiment of the apparatus for producing a cell-processed product of the present disclosure. Each of the apparatuses 1 and 1a for producing a cell-processed product as illustrated in FIGS. 1-1 and 1-2 includes a liquid M as the pressure wave medium, a pressure-resistant container 11 configured to accommodate a cell, and a pressure wave application unit 13 configured to apply the pressure wave into the pressure-resistant container 11 through the liquid M with the predetermined pressure profile.

The heat-resistant container 11 is typically a cylinder formed of metal, and in the apparatus 1 for producing a cell-processed product as illustrated in FIG. 1-1, the inside of the heat-resistant container 11 is sealed with a lid 12 detachably attached to the upper end opening. As in the apparatus 1a for producing a cell-processed product as illustrated in FIG. 1-2, the lid 12 may not be included. In the heat-resistant container 11, the liquid M as the pressure wave medium is accommodated, and a container 3 containing a first liquid S with the cell is accommodated so as to be immersed in a second liquid M as the pressure wave medium.

In the apparatus 1 for producing a cell-processed product, an actuator (pressure wave application unit) 13 configured to accommodate a pyrotechnic charge 2 can be fixed to the back side of the lid 12 of the pressure-resistant container 11 (the inner side of the pressure-resistant container 11). In the apparatus 1a for producing a cell-processed product, the actuator (pressure wave application unit) 13 configured to accommodate the pyrotechnic charge 2 can be suspended from above. The pyrotechnic charge 2 includes an igniter (i.e., initiator) in which an ignition charge is sealed with an outer shell made of, for example, metal. A housing included in the actuator 13 is provided so that the inside of the housing and the inside of the pressure-resistant container 11 do not communicate with each other, and in the actuator 13, a pressure relief channel 131 configured to guide explosive gas generated in the housing into the pressure-resistant container 11, and a breakable portion 132 configured to be breakable by the explosive gas, as the outlet of the pressure relief channel 131 are provided in a wall, specifically, the bottom wall in contact with the inside of the pressure-resistant container 11. The breakable portion 132 may have any form as long as, in the bottom wall, it is formed to be more fragile than the other walls. In the apparatuses 1 and 1a for producing a cell-processed product, as an embodiment of the pressure relief channel 131 and the breakable portion 132, an embodiment is exemplified in which the pressure relief channel 131 is recessed in the bottom wall and thus, the breakable portion 132 having a reduced thickness is formed.

When each of the apparatuses 1 and 1a for producing a cell-processed product is operated, an electrical signal supplied from a power source is applied to the initiator in the pyrotechnic charge 2, and thus, the ignition charge loaded in the initiator is ignited. As the temperature inside the initiator becomes high due to heat generated by the igniting, the outer shell of the initiator is opened, the breakable portion 132 of the actuator 13 is subsequently blasted, a plasma-like flame emitted from the pressure relief channel 131 compresses the pressure medium to generate a pressure wave, which propagates through the pressure wave medium M, the predetermined pressure profile is applied to the cell contained in the liquid S accommodated in the container 3, and partial damage is inflicted on the surface layer of the cell. The predetermined pressure profile is as described in "1-5. Pressure Profile".

Each aspect disclosed in the present specification can be combined with any other feature disclosed in the present specification.

### Examples

Hereinafter, the present invention will be more specifically described by Examples, but each of the configurations, combinations thereof, and the like in each of the embodiments are merely examples, and additions, omissions, substitutions, and other changes of the configurations may be made as appropriate without departing from the spirit of the present invention. The present disclosure is not limited by the embodiments and is limited only by the claims.

### [1] Test Design

The following cell processing was performed.

**[Table 1]**

| | Cells Used | Cell Processing | Type of Processing |
|---|---|---|---|
| Comparative Example 1a-1 | S. cerevisiae strain a924E1 | No processing (control), only FITC addition | |
| **Example 1** | **S. cerevisiae strain a924E1** | **Pressure wave application** | **FITC introduction** |
| Comparative Example 1a-2 | S. cerevisiae strain a924E1 | No processing (control), Only FITC-dextran4 addition | |
| **Example 2** | **S. cerevisiae strain a924E1** | **Pressure wave application** | **FITC-dextran4 introduction** |
| Comparative Example 1a-3 | S. cerevisiae strain a924E1 | No processing (control), Only PI addition | |
| **Example 3** | **S. cerevisiae strain a924E1** | **Pressure wave application** | **PI introduction** |
| Comparative Example 1b | S. cerevisiae strain YPH499 | No processing (control) | |
| **Example 4a** | **S. cerevisiae strain YPH499** | **Pressure wave application** | **Extraction** |
| Comparative Example 2 | S. cerevisiae strain YPH499 | Electroporation | Extraction |
| Comparative Example 1c | S. cerevisiae strain ATCC9763 | No processing (control) | |
| **Example 4b** | **S. cerevisiae strain ATCC9763** | **Pressure wave application** | **Extraction** |
| Comparative Example 3a | S. cerevisiae strain ATCC9763 | Pulsed electric field processing (10 kV/cm, 1.4 µs) | |
| Comparative Example 3b | S. cerevisiae strain ATCC9763 | Pulsed electric field processing (20 kV/cm, 1.4 µs) | |
| Comparative Example 3c | S. cerevisiae strain ATCC9763 | Pulsed electric field processing (30 kV/cm, 1.4 µs) | |
| Comparative Example 3d | S. cerevisiae strain ATCC9763 | Pulsed electric field processing (40 kV/cm, 1.4 µs) | |
| Comparative Example 3e | S. cerevisiae strain ATCC9763 | Pulsed electric field processing (50 kV/cm, 1.4 µs) | |
| Comparative Example 4 | S. cerevisiae strain ATCC9763 | Pulsed electric field processing (100 kV/cm, 130 ns) | |

### [2] Test Procedure (Examples 1 to 3)

### [2-1] Preparation of Cells

Budding yeast (S. cerevisiae strain a924E1) was inoculated into YPD liquid culture medium and cultured with shaking at 30°C for 16 hours. The resulting cultured liquid was centrifuged at 4°C to sediment the cells, the supernatant was discarded, and the cells were washed with PBS. (Hereinafter, a series of operations including centrifugation at 4°C, cell sedimentation, supernatant removal, and washing with PBS is referred to as "centrifugation and cell washing".)

### [2-2] Preparation of Cell-Containing Liquid

The cells obtained in [2-1] above were diluted with saline, and the concentration of the cells was adjusted to about 10 × 10⁸ cells/mL per sample to yield a cell-containing liquid. To the resulting cell-containing liquid, 500 µM of FITC, 500 µM of FITC-dextran4 (4 KDa, FITC-labeled dextran having a weight-average molecular weight of 4000), or 74.8 µM of PI was added, respectively as fluorescent substances. Each of the cell-containing liquids was sealed in an FEP microtube with a volume of 150 µL (manufactured by Pressure bioscience, Inc.) at 150 µL per microtube. Separately, cell-containing liquids as controls (Comparative Examples 1a-1 to 1a-3) were stored on ice. Note that, in all of the Examples and Comparative Examples, each of the cell-containing liquids was in the form of a cell suspension in which the cells were uniformly suspended.

### [2-3] Cell Processing (Pressure Wave Application)

The apparatus 1 for producing a cell-processed product illustrated in FIG. 1-1 was prepared. In the cell-processed product production assumption 1 used, the distance from the breakable portion 132 to the upper end of the container 3 was about 15 mm, the distance from the breakable portion 132 to the bottom of the container 3 was about 39 mm, and the volume of the heat-resistant container 11, which was a metal cylinder, was 10 ml. A microtube in which each of the cell-containing liquids was sealed was placed in the heat-resistant container 11, and the heat-resistant container 11 was filled with water, and thus, the microtube was immersed. As an explosive contained in the pyrotechnic charge 2 accommodated in the actuator (pressure wave application unit) 13 attached to the lid 12, 190 mg of ZPP (in a form sealed with a stainless-steel outer shell) was used. The heat-resistant container 11 was sealed by covering the lid 12.

An electrical signal was applied to the initiator, the ignition charge was ignited, and thus, dynamic pressure was applied to the sample tube. Specific pressure profile was as follows. Note that the following pressure profile was measured at a location separated from the breakable portion 132 corresponding to the pressure application unit, by about 1.5 times the shortest distance between the breakable portion 132 and the location where the cell exists in FIG. 1-1 (specifically, the location indicated by a triangle mark).
(i) Maximum value of pressure: from 148 to 266 MPa
(ii) Time from start of the pressure rise to a time point when the pressure reaches the maximum value: from 0.04 to 0.12 ms
(iii) Time from start of the pressure decay from the maximum value to a time point when the pressure decays to 1/2 of the maximum value: from 0.2 to 0.3 ms

The sample tube was removed from the sealed container. This operation was repeated 16 times.

### [2-4] Evaluation of Fluorescent Substance Introduction

Centrifugation and cell washing were performed on the unprocessed cell-containing liquids (control; Comparative Examples 1a-1 to 1a-3), and the cell-containing liquids subjected to the processing described in [2-3] above (Examples 1 to 3). Subsequently, the cells were observed with a phase-contrast microscope (BX41; Olympus Corporation), and the fluorescence intensity was observed with a fluorescence plate reader (Gemini XPS; manufactured by MOLECULAR DEVICES, LLC.) or evaluated with a flow cytometer (Cell Sorter SH800S; manufactured by Sony Corporation).

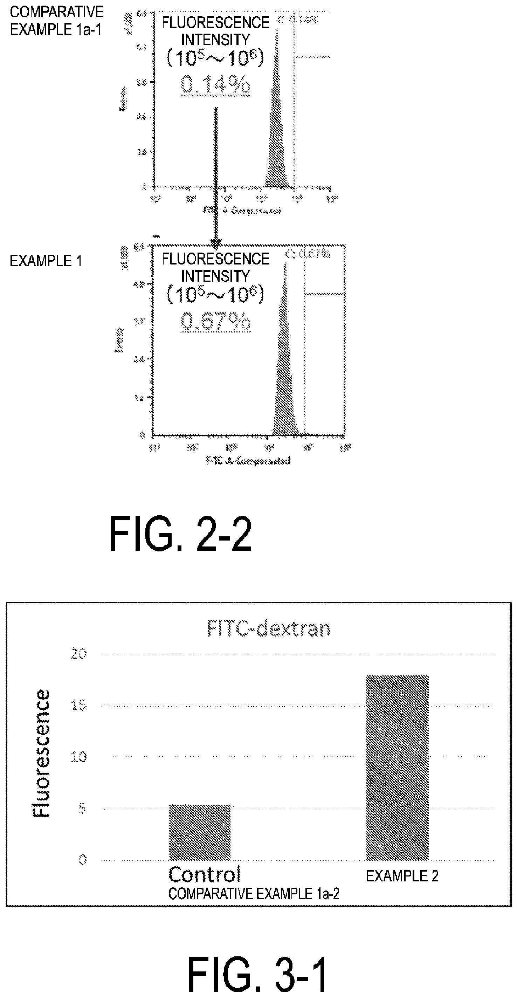

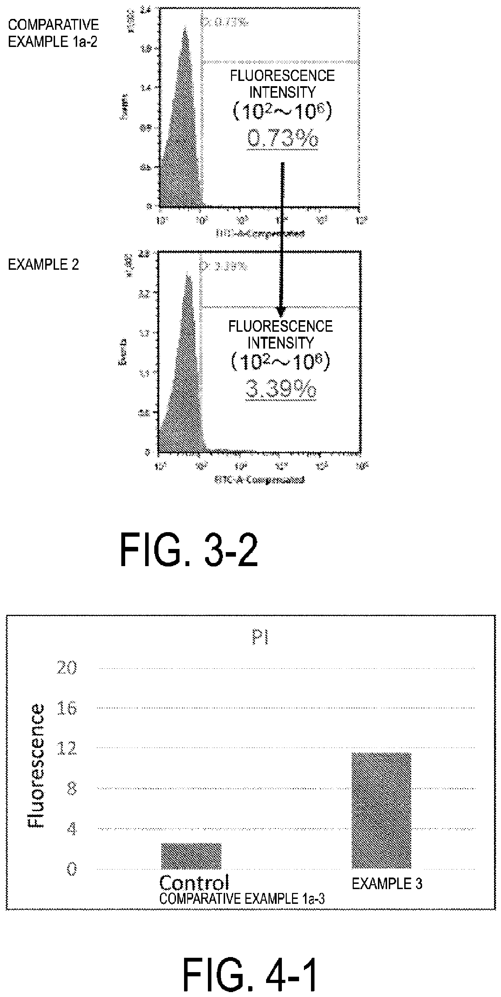

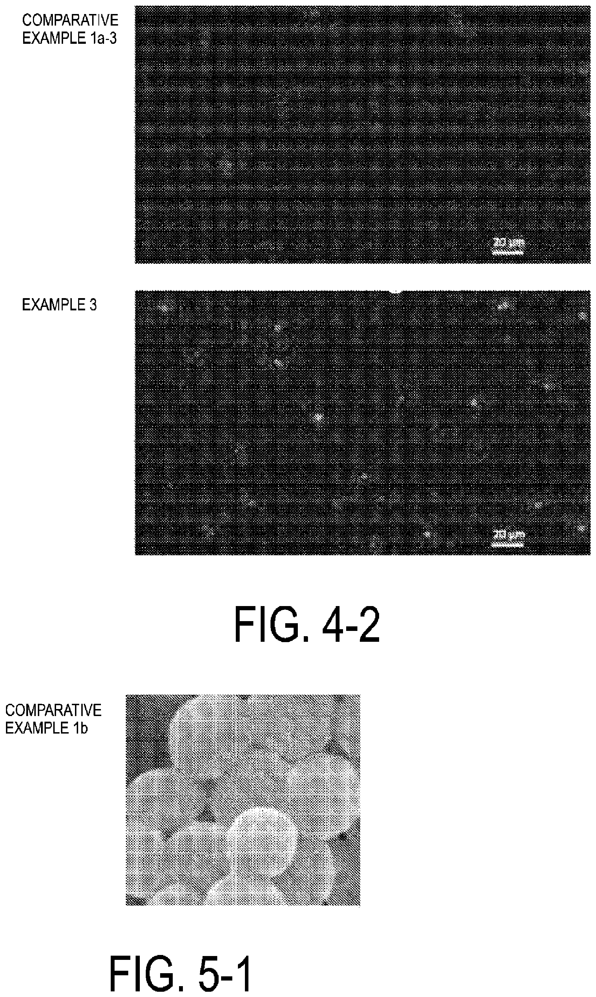

For Comparative Example 1a-1 and Example 1, the evaluation results by the fluorescence plate reader are shown in FIG. 2-1, and the evaluation results by the flow cytometer are shown in FIG. 2-2. For Comparative Example 1a-2 and Example 2, the evaluation results by the fluorescence plate reader are shown in FIG. 3-1, and the evaluation results by the flow cytometer are shown in FIG. 3-2. For Comparative Example 1a-3 and Example 3, the evaluation results by the fluorescence plate reader are shown in FIG. 4-1, and the fluorescence microscope images are shown in FIG. 4-2. As shown by these results, it was confirmed that the fluorescent substances were introduced in Examples 1 to 3.

### [3] Test Procedure (Example 4a, Comparative Example 2)

### [3-1] Preparation of Cells and Cell-Containing Liquid

Budding yeast (S. cerevisiae strain YPH499) was inoculated into YPD liquid culture medium and cultured with shaking at 30°C for 8 hours. The resulting cultured liquid was centrifuged at 4°C to sediment the cells, the supernatant was discarded, and the cells were washed with PBS. Next, the cells were dispersed in sterile water, and centrifuged again to sediment the cells. The supernatant was discarded, and the sedimented cells were suspended by adding an aqueous 1M sorbitol solution. Then, the suspension was centrifuged to sediment the cells. The supernatant was discarded, and the sedimented cells were suspended by adding an aqueous 1M sorbitol solution to yield a cell-containing liquid. Separately, a cell-containing liquid as a control (Comparative Example 1b) was stored on ice.

### [3-2] Cell Processing (Pressure Wave Application, Example 4a)

The cell-containing liquid obtained in [3-1] above was sealed in an FEP microtube (manufactured by Pressure bioscience, Inc.) in an amount of 150 µL. Next, the cell-containing liquid was subjected to pressure wave application processing in the same manner as in [2-3] above.

### [3-3] Electroporation Processing (Comparative Example 2)

The cell-containing liquid obtained in [3-1] above was added to a 0.2 cm cuvette with electrodes in an amount of 40 µL, and the cell-containing liquid was subjected to electroporation using Gene Pulser Xcell under the conditions of 5 ms width and 1500 V. Immediately after the electroporation, 1 mL of an aqueous 1M sorbitol solution was added to dilute the cell-containing liquid.

### [3-4] Scanning Electron Microscope (SEM) Observation

Centrifugation and cell washing were performed on the unprocessed cell-containing liquid (control; Comparative Example 1), and the cell-containing liquids (Example 4a, Comparative Example 2) subjected to the processing of [3-2] or [3-3] above, and the cells were fixed with glutaraldehyde. After the fixation, the cells were washed again, dehydrated with alcohol, immersed in t-butyl alcohol, lyophilized, and subjected to gold deposition to prepare samples for SEM observation. The surface layer of the yeast cells was observed using SEM.

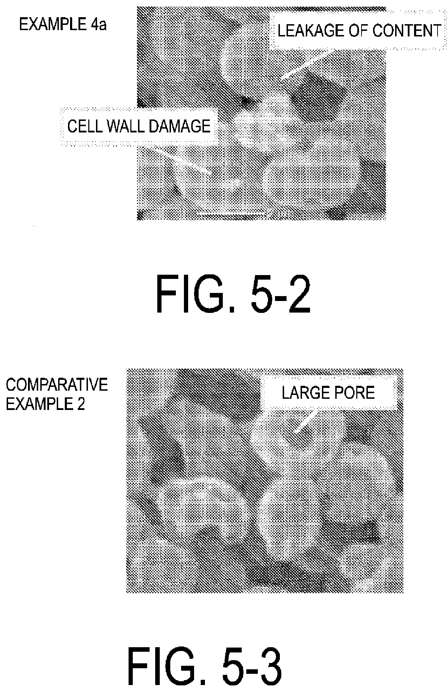

The SEM images of Comparative Example 1b, Example 4a, and Comparative Example 2 are shown in FIGS. 5-1, 5-2, and 5-3, respectively. As shown in FIG. 5-2, according to Example 4a, slight damage to the cell walls and leakage of the content were observed, and the degree of damage to the cell walls was significantly suppressed as compared with the degree of damage to the cell walls (formation of large pores) according to Comparative Example 2 (electroporation) shown in FIG. 5-3.

### [4] Test Procedure (Example 4b, Comparative Examples 3a to 3e)

### [4-1] Preparation of Cells

Budding yeast (S. cerevisiae strain ATCC 9763) was inoculated into YPD liquid culture medium and cultured with shaking at 30°C for 24 hours. The resulting cultured liquid was subjected to centrifugation and cell washing.

### [4-2] Preparation of Cell-Containing Liquid

The cells obtained in [4-1] above were diluted with D-PBS, and the concentration of the cells was adjusted to about 10 × 10⁹ cells/mL per sample to yield a cell-containing liquid. Separately, a cell-containing liquid as a control (Comparative Example 1c) was stored on ice.

### [4-3] Cell Processing (Pressure Wave Application, Example 4b)

The cell-containing liquid obtained in [4-2] above was sealed in an FEP microtube (manufactured by Pressure bioscience, Inc.) in an amount of 150 µL. Next, the cell-containing liquid was subjected to pressure wave application processing in the same manner as in [2-3] above.

### [4-4] Cell Processing (Pulsed Electric Field Processing, Comparative Examples 3a to 3e)

The cell-containing liquid was introduced between stainless-steel parallel-plate electrodes with a gap of 3 mm. Then, a microsecond pulse with a pulse width of 1.4 µs and an electric field of 10 to 50 kV/cm was applied 100 times at 1 Hz to each of Comparative Examples 3a to 3e. At this time, the pulse was applied, while the electrodes were water-cooled to ensure that the temperature of the cell-containing liquid did not exceed 35°C.

### [4-5] Measurement of Extraction Amount

The cell-containing liquid of the control (Comparative Example 1c) and the cell-containing liquids (Example 4b, Comparative Examples 3a to 3e) subjected to the processing of [4-3] or [4-4] above were centrifuged to yield the supernatants. Each of the supernatants was filtered with a 450 nm membrane filter, and then subjected to electrophoresis using a 10-20% gradient gel at a voltage of 300 V for 4 hours, followed by CBB staining and imaging.

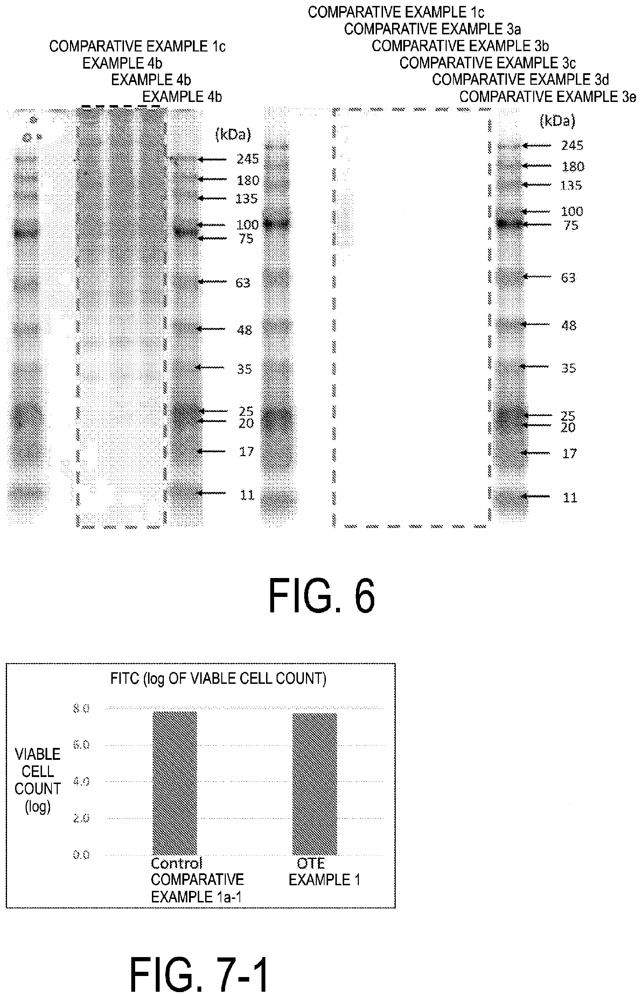

The electrophoretic image is shown in FIG. 6. The amount of protein extracted in Example 4b was significantly increased as compared with each of the amounts of protein extracted in Comparative Examples 3a to 3e (pulsed electric field processing).

### [5] Evaluation of Viable Cell Count

### [5-1] Evaluation of Viable Cell Count of Processed Cells subjected to Introduction to Cells by Pressure Wave Application

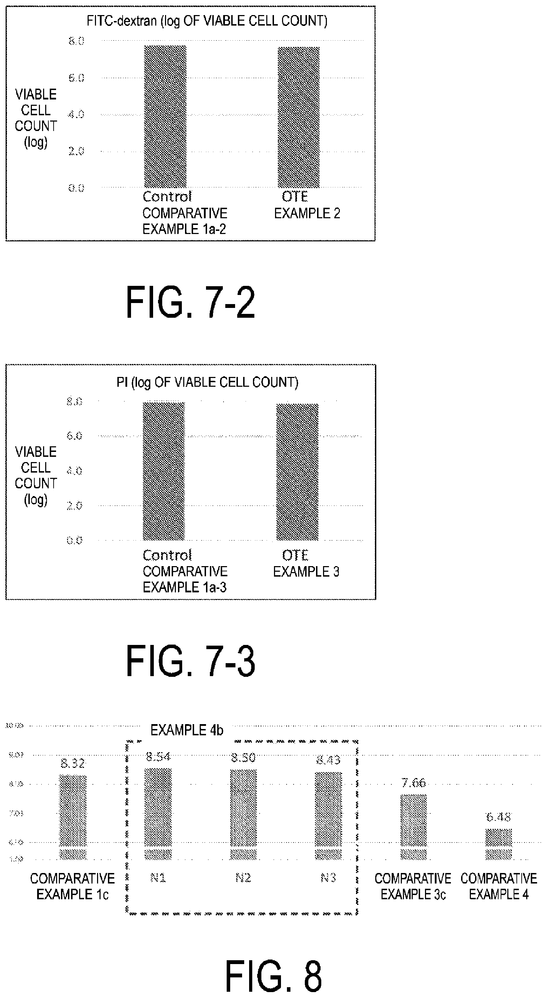

Each of the processed cells obtained in Comparative Examples 1a-1 to 1a-3 and Examples 1 to 3 were centrifuged, washed, serially diluted, inoculated on YPD agar medium, and cultured at 30°C for 120 hours. Thereafter, colony counting was performed by visual observation. The results are shown in FIGS. 7-1 to 7-3.

As shown in FIGS.7-1 to 7-3., it was confirmed that the cells were not killed in the cell processing according to Examples 1 to 3.

### [5-2] Evaluation of Viable Cell Count of Processed Cells subjected to Extraction by Pressure Wave Application and Processed Cells Subjected to Pulse Electrolysis Processing

First, the pulsed electric field processing was performed in the same manner as in [4-4] above except that the conditions of the pulsed electric field processing were changed to nanosecond pulses with a pulse width of 200 ns and an electric field of 100 kV/cm (Comparative Example 4).

Each of the processed cells obtained in Comparative Example 1c, Example 4, Comparative Example 3c, and Comparative Example 4b were centrifuged, washed, serially diluted, inoculated on YPD agar medium, and cultured at 30°C for 36 hours. Thereafter, colony counting was performed by visual observation. The results are shown in FIG. 8.

As shown in FIG. 8, it was confirmed that the cells were killed in Comparative Examples 3c and 4 (pulsed electric field processing), whereas the cells were not killed in the cell processing according to Example 4b.

### Reference Signs List

1, 1a Apparatus for producing cell-processed product
11 Heat-resistant container
12 Lid
13 Pressure wave application unit (actuator)
131 Pressure relief channel
132 Breakable portion
2 Pyrotechnic charge
3 Container (container containing first liquid S with cell)
M Liquid as pressure wave medium (second liquid)
S First liquid with cell

## Claims

1. A method for producing a cell-processed product, the method comprising processing a cell by applying a pressure wave to the cell using a liquid as a pressure wave medium, and thus, inflicting partial damage on a surface layer of the cell, wherein
a pressure profile of the pressure wave includes conditions (i) to (iii):
(i) a maximum value of the pressure is 37 MPa or greater and 500 MPa or less;
(ii) time from start of the pressure rise to a time point when the pressure reaches the maximum value is 0.5 ms or less; and
(iii) time from start of the pressure decay from the maximum value to a time point when the pressure decays to 1/2 of the maximum value is 5 ms or less.

2. The production method according to claim 1, wherein
the pressure wave is applied to a container containing a first liquid with the cell, using a second liquid as the pressure wave medium, the container being immersed in the second liquid.

3. The production method according to claim 1, wherein
the cell coexists with a molecule to be introduced into the cell, and in the processing the cell, the molecule is introduced into the cell through the partial damage; and
the cell-processed product is the cell containing the introduced molecule.

4. The production method according to claim 3, wherein
the molecule is selected from the group consisting of a cell-staining dye, a drug delivery carrier, and a drug delivery carrier labeled with the cell-staining dye.

5. The production method according to claim 1, wherein
in the processing the cell, at least a part of a cytoplasmic component of the cell is extracellularly released through the partial damage; and
the cell-processed product is a cell lacking the at least a part of the cytoplasmic component and/or the at least a part of the cytoplasmic component.

6. The production method according to claim 1, wherein
the application is repeatedly operated in the processing the cell.

7. An apparatus for producing a cell-processed product, the apparatus comprising:
a liquid as a pressure wave medium;
a pressure-resistant container configured to accommodate a cell; and
a pressure wave application unit configured to apply a pressure wave into the pressure-resistant container through the liquid with a pressure profile, the pressure profile including conditions (i) to (iii):
(i) a maximum value of the pressure is 37 MPa or greater and 72500 psi or less;
(ii) time from start of the pressure rise to a time point when the pressure reaches the maximum value is 0.5 ms or less; and
(iii) time from start of the pressure decay from the maximum value to a time point when the pressure decays to 1/2 of the maximum value is 5 ms or less.

8. The production apparatus according to claim 7, wherein
the pressure wave application unit is not loaded with a gas generating agent.
